# EUROPEAN PATENT SPECIFICATION

(11) **EP 1 666 010 B1**
(45) Date of publication and mention of the grant of the patent: **16.01.2008**
(21) Application number: 05257064.5
(22) Date of filing: 16.11.2005
(51) Int. Cl.: A61F 13/15

(54) **Absorbent core forming suction drum**
Vakuumtrommel zur Formung von Absorptionskörpern
Collecteur cylindrique de formage des noyaux absorbants

(30) Priority: 18.11.2004 JP 2004334282
(43) Date of publication of application: 07.06.2006
(73) Proprietor: UNI-CHARM CORPORATION, Shikokuchuo-shi, Ehime 799-0111 (JP)
(72) Inventor: Otsubo, Toshifumi, c/o Technical Center, Mitoyo-gun Kagawa-ken 769-1602 (JP)
(74) Representative: Fitchett, Stuart Paul

(56) References cited:
- US-A- 3 939 240
- US-A- 4 741 941
- US-A- 5 665 396

## Description

The present invention relates to a liquid-absorbent material molding drum for making liquid-absorbent materials to be incorporated in disposable body fluid absorbent articles such as disposable diapers, sanitary napkins or pads for continent patient.

There have already been proposed liquid-absorbent core molding drums comprising an annular peripheral surface extending in a circumferential direction, a plurality of molding depressions formed on the peripheral surface and distanced one from another by a predetermined dimension in the circumferential direction and a suction mechanism operatively associated with each of the molding depressions in such a manner that the suction mechanism sucks air through a plurality of fine vent holes distributed over an entire area of the depression's bottom. Such a molding drum is disclosed, for example, in Reference, i.e., Japanese Unexamined Patent Application Publication No. 2002-272782. The drum rotates around its shaft. An air outlet and a compression drum are provided so as to be opposed to the peripheral surface of this drum. The duct is provided at its air inlet with a grinder mill adapted to crush into pulp sheets into crushed pulp/fluff pulp and, in addition, the duct is provided at its longitudinal middle with a hopper adapted to pour super-absorbent polymer particles into the duct.

A sequence in which the liquid-absorbent core is made using this known molding drum is as follows: A pulp sheet is guided by guide rolls into the grinder mill and crushed therein into fluff pulp which is then introduced into the duct. At the same time, the polymer particles are supplied from the hopper into the duct. Within the duct, the suction mechanism operatively associated with the molding drum sucks air so as to flow from the inlet toward the outlet of the duct. The fluff pulp and the polymer particles are fanned in drift within the duct and conveyed by air stream toward the outlet. In the course of conveyance, the fluff pulp and the polymer particles are agitated together to form the mixture thereof. The mixture is blown by the air stream from the outlet of the duct against the peripheral surface of the molding drum. The molding depressions are periodically opposed to the outlet of the duct as the drum rotates around the shaft whereupon the mixture is collected and accumulated in one of the molding depressions under the effect of the suction mechanism operatively associated with the molding drum to mold the mixture into a shape of the one. The mixture molded in the molding depression is held in this molding depression under sucking force of this suction mechanism until the molded mixture reaches to a compressed drum as the molding drum rotates. Thereupon, the molded mixture is transferred from the peripheral surface of the molding drum to a peripheral surface of the compression drum under sucking force of a suction mechanism operatively associated with the compression drum. The molded mixture is then compressed between the peripheral surface of the compression drum and a running conveyor to a predetermined thickness to form an absorbent core. The absorbent core is then transferred from the peripheral surface of the compression drum onto the conveyor under sucking force of a suction mechanism provided below the conveyor. The liquid-absorbent core make in this manner is incorporated in disposable body fluid absorbent articles such as disposable diapers, sanitary napkins or pads for incontinent patient.

In the case of the molding drum disclosed in Reference, the mixture of fluff pulp and polymer particles is fanned in drift by air stream and blown against the peripheral surface of the drum. In this step, differential specific gravity of fluff pulp and polymer particle and/or turbulence of the air stream may make it difficult to accumulate the mixture evenly in the entire surface of the molding depression.

Consequently, the mixture may be collected in a part of the molding depression in a concentrated manner and the molding depression may be locally formed with a region in which the mixture presents a relatively high density and basis weight. If the mixture having such region of high density and basis weight is compressed as it is, the finished liquid-absorbent core will have a locally high stiff region. In other words, it will be impossible not only to make the liquid-absorbent core having a uniform stiffness in its entirety but also to make the liquid-absorbent core which is flexible in its entirety. In the case of the liquid-absorbent core locally formed with the stiff region, body fluids will be absorbent in such stiff region in a concentrated manner under capillary phenomenon significantly occurring in this region and it will be not expected that body fluids can be effectively diffused over the whole area of the liquid-absorbent core. Thus it may be impossible to utilize the liquid-absorbent core in its entirety for absorption of body fluids.

Prior art liquid-absorbent material moulding drums are known from US 5665396 and US 3939240. US 3939240 discloses a moulding drum comprising a peripheral surface, a plurality of moulding depressions formed on said peripheral surface, a suction mechanism, and a plurality of protrusions formed on a bottom of each of the moulding depressions. US 5665396 discloses a similar arrangement that features a single moulding depression.

In view of the problem as has been described above, it is an object of the present invention to provide a liquid-absorbent core molding drum allowing it to make the liquid-absorbent core having a uniform stiffness as well as a high flexibility and being able to absorb body fluids efficiently over its whole area.

According to the present invention, there is provided a liquid-absorbent material molding drum comprising: a peripheral surface extending in a circumferential direction, a plurality of molding depressions formed on said peripheral surface; a suction mechanism adapted to suck air inward with respect to said circumferential direction from vent holes formed in a bottom of said molding drum; and said molding depression being formed on said bottom with a plurality of protrusions, each protruding outward in a diametrical direction of said molding drum and being longest in said circumferential direction of said molding drum, said plurality of protrusions being continuously or intermittently arranged in said circumferential direction but distanced one from another by a predetermined dimension in an axial direction of said molding drum, characterized in that a height dimension of an apex region of each protrusion as measured from the bottom of said molding depression is larger than a height dimension as measured from the bottom of said molding depression to said peripheral surface, such that said apex region protrudes outward beyond said peripheral surface.

The present invention may include preferred in manners embodiments as follows:

The apex region of each of the protrusions lies in a longitudinal middle of the protrusion, a first region lying in front of the apex region as viewed in the circumferential direction and defining a slope obliquely extending from the apex region toward the bottom of the molding depression, and a second region lying behind the apex region as viewed in the circumferential direction and defining a slope obliquely extending from the apex region toward the bottom of the molding depression.

In each pair of the protrusions adjacent to each other in the circumferential direction, the first region of the one protrusion is opposed to the second region of the other protrusion and, in each pair of the protrusions adjacent to each other in the axial direction, the first region of the one protrusion is opposed to the second region of the other protrusion.

The protrusion describes a semi-circular arc being convex in the axial direction.

Each pair of the protrusions being adjacent to each other in the circumferential direction describe the semi-circular arcs which are convex alternately in axially opposite directions.

With the liquid-absorbent material molding drum according to the present invention, the absorbent material is accumulated in the molding depression except the protrusions and the absorbent material may be locally accumulated in the molding depression in concentrated manner. However, even if high density zones in which a density of the absorbent material is relatively high are locally formed in the molding depression, such high density zones are segmented by the protrusions and the high density zones can be segmented by the protrusions and a density of these high density zones is effectively reduced by the protrusions. The absorbent material molded using this molding drum has a plurality of the patterned zones formed by the protrusions so as to be intermittently arranged, so these high stiffness zones are segmented by the patterned zones and a stiffness of the high stiffness zones is reduced by these patterned zones. The high stiffness zones segmented by the patterned zones allow body fluids to be absorbed by the absorbent material over its whole area rather than being absorbed by the high stiffness zones in concentrated manner. In this way, this molding drum is able to make the absorbent material having a generally uniform stiffness and high flexibility so that body fluids can be efficiently absorbed by the absorbent material over its whole area.
Fig. 1 is a perspective view showing an apparatus for making liquid-absorbent materials including a materials molding drum according to a first embodiment of the invention;
Fig. 2 is a sectional side view showing a duct and the molding drum constituting the apparatus for making the liquid-absorbent materials;
Fig. 3 is a perspective partial view showing, in an enlarged scale, a peripheral surface and molding depression of the molding drum;
Fig. 4 is a sectional view taken along the line 4-4 in Fig. 3;
Fig. 5 is a plan view showing the liquid-absorbent material made using the molding drum shown in Fig. 1;
Fig. 6 is a plan view showing a part of Fig. 5 in an enlarged scale;
Fig. 7 is a sectional view taken along the line 7-7 in Fig. 6;
Fig. 8 is a partially cutaway plan view showing a disposable diaper including the material shown in Fig. 1;
Fig. 9 is a perspective partial view showing, in an enlarged scale, a peripheral surface and molding depression of the molding drum according to a first example that does not constitute an embodiment of the invention;
Fig. 10 is a sectional view taken along the line 10-10 in Fig. 9;
Fig. 11 is a plan view showing the liquid-absorbent material made using the molding drum shown in Fig. 9;
Fig. 12 is a plan view showing a part of Fig. 11 in an enlarged scale; and
Fig. 13 is a sectional view taken along the line 13-13 in Fig. 12.

Details of a liquid-absorbent material molding drum according to the present invention will be more fully understood from the description given hereunder with reference to the accompanying drawings.

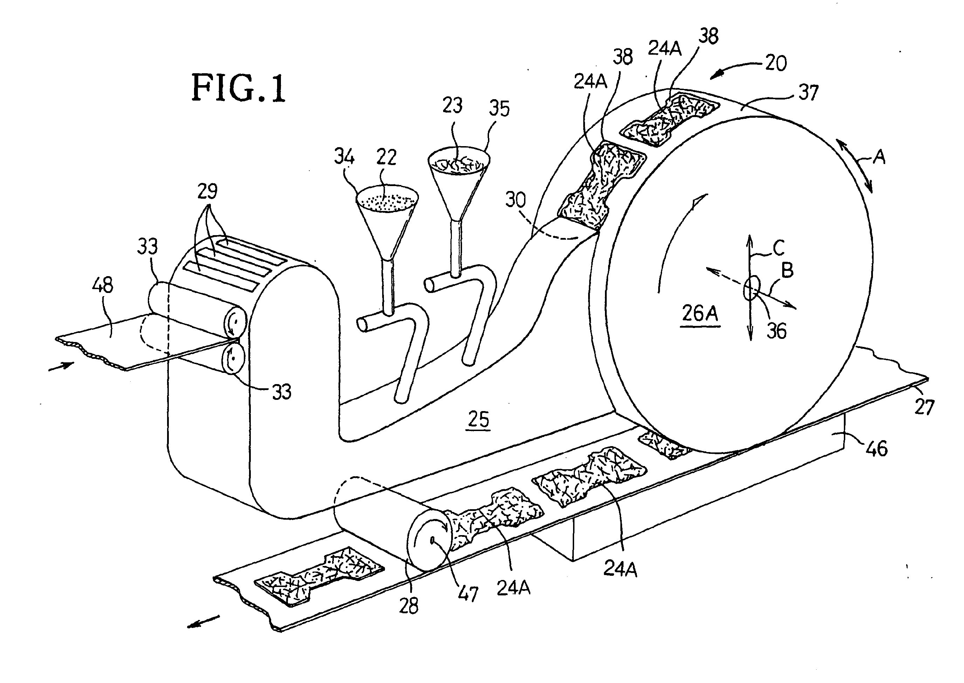

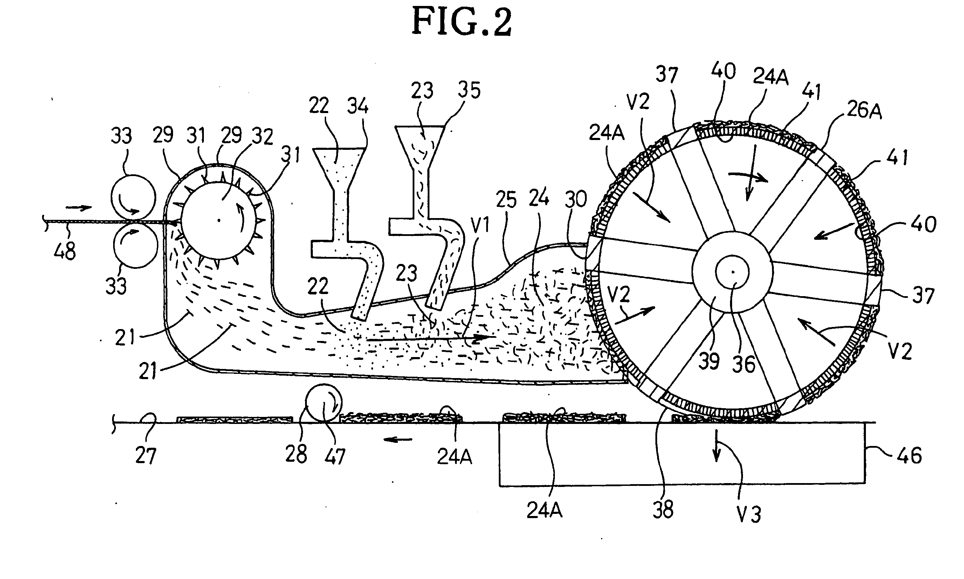

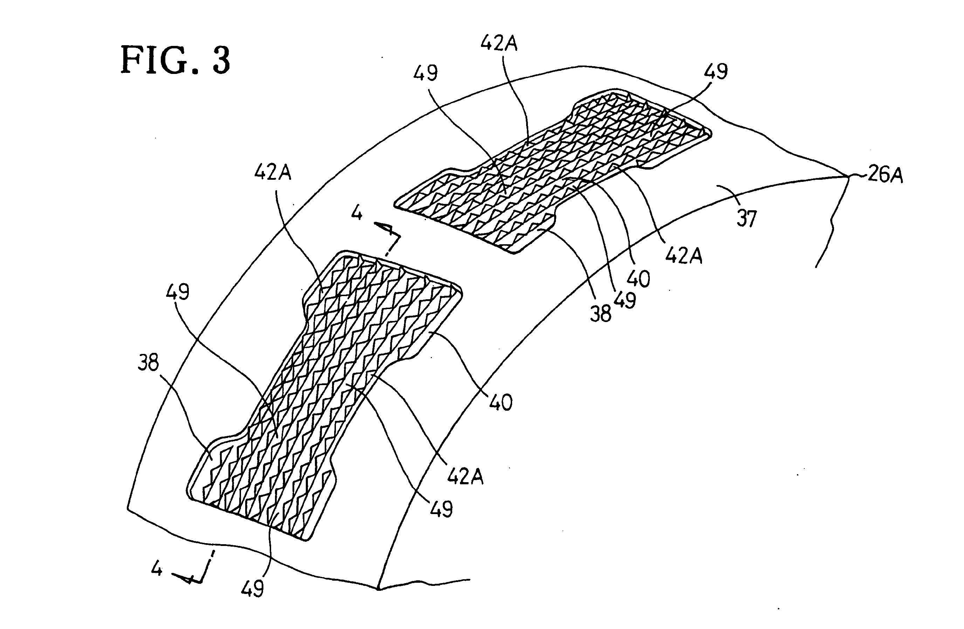

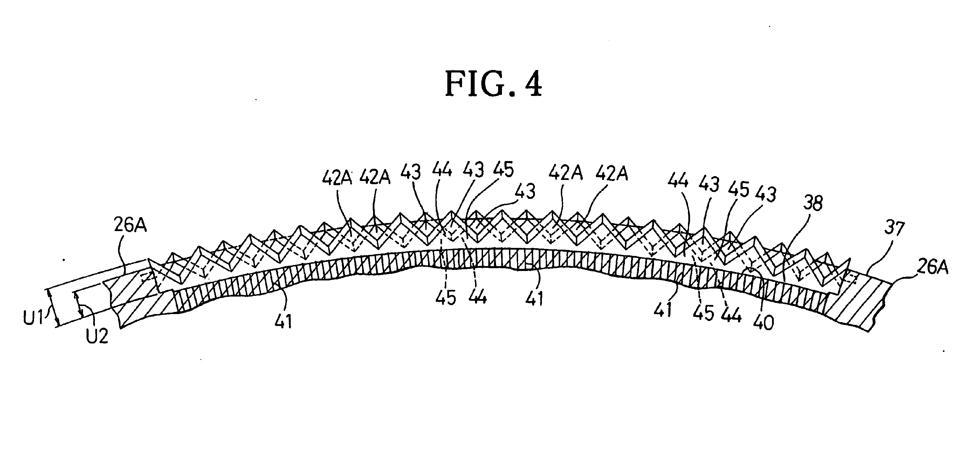

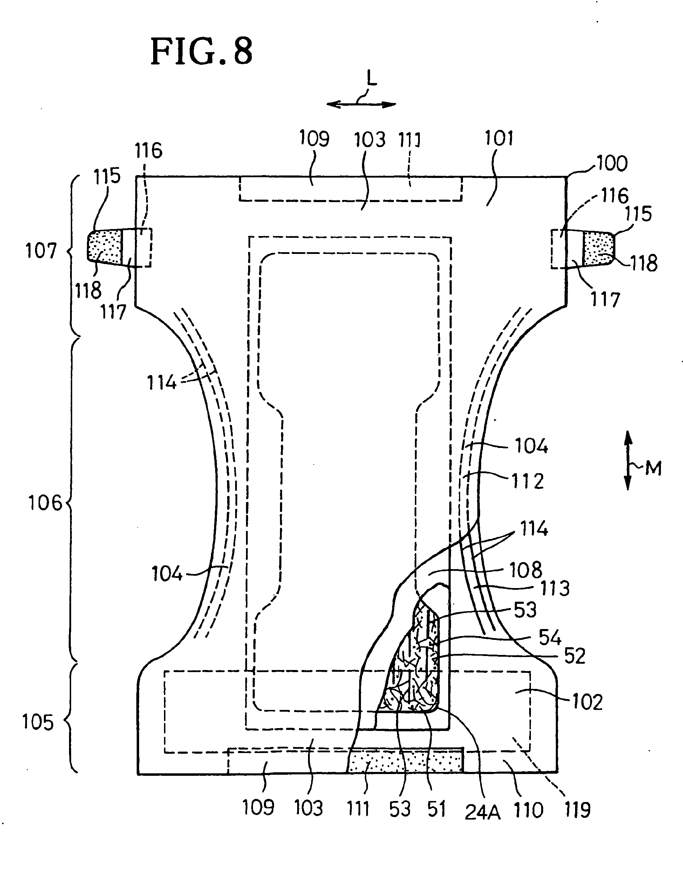

Fig. 1 is a perspective view showing an apparatus 20 for making liquid-absorbent materials including a material molding drum 26A, Fig. 2 is a sectional side view showing a duct 25 and the molding drum 26A constituting the apparatus 20, Fig. 3 is a perspective partial view showing, in an enlarged scale, a peripheral surface 37 and molding depression 38 of the molding drum 26A and Fig. 4 is a sectional view taken along a line 4-4 in Fig. 3. In Fig. 1, a circumferential direction of the drum 26A is indicated by an arrow A, and an axial direction of the drum 26A is indicated by an arrow B and a diametrical direction of the drum 26A is indicated by an arrow C. In Figs. 1 and 2, a direction in which untreated pulp 48 is fed, directions in which grinder mill 32, the drum 26A, a compression drum 28 and guide rollers 33 rotate and a direction in which conveyor 27 runs are indicated by arrows, respectively. This apparatus 20 including the molding drum 26A is adapted to make the liquid-absorbent material used as an absorbent core in the disposable body fluid absorbent article such as disposable diaper 100 (See Fig. 8), sanitary napkin or incontinent pad.

The apparatus 20 comprises the duct 25 adapted to prepare a mixture 24 of fluff pulp 21, super-absorbent polymer particles 22 and thermoplastic synthetic resin fibers 23, the liquid-absorbent material molding drum 26A adapted to mold the mixture 24 into a liquid absorbent material 24A such as, example, a liquid-absorbent core having a generally hourglass shape, the conveyor 27 adapted to receive the absorbent material 24A molded from the drum 26A, and the compression drum 28 adapted to compress the absorbent material 24A to a predetermined thickness. The duct 25 extending toward the drum 26A has an air inlet 29 and an air outlet 30. The inlet 29 of the duct 25 is provided with the grinder mill 32 having a plurality of blades 31 and a pair of the guide rollers 33 opposed to each other. The duct 25 is provided at intermediate regions thereof as viewed in a longitudinal direction of the duct 25 with a hopper 34 adapted to supply super-absorbent polymer particles 22 into the duct 25 and a hopper 35 adapted to supply thermoplastic synthetic resin fibers 23 into the duct 25. The outlet 30 of the duct 25 is opposed to a peripheral surface 37 of the drum 26A. The peripheral surface 37 will be described later in more detail. The grinder mill 32 and the guide rollers 33 rotate in directions respectively indicated by arrows.

The molding drum 26A comprises the shaft 36, the annular peripheral surface 37 extending in the circumferential direction of the drum 26A and a plurality of the molding depressions 38 formed on the peripheral surface 37. The drum 26A is rotated by the shaft 36 in the direction indicated by the arrow. Within the drum 26A, there is provided a suction mechanism 39 adapted to suck air from the molding depressions 38 toward the interior of the drum 26A. The molding depressions 38 are distanced one from another by a predetermined dimension in the circumferential direction, each of these molding depressions 38 is shaped in a generally hourglass which is relatively long in the circumferential direction of the drum 26A and depressed from the peripheral surface 37 toward the interior of the drum 26A. The molding depression 38 is formed over a whole area of its bottom with a plurality of fine slit-like vent holes 41 (See Figs. 2 and 4) and a plurality of protrusions 42A rectilinearly extending in the circumferential direction of the drum 26A. Each of the vent holes 41 is dimensioned to be permeable for air but not for the mixture 24.

Each of the protrusions 42A is relatively long in the circumferential direction of the drum 26A and protrudes outward in a diametrical direction of the drum 26A from a bottom 40 of the molding depression 38. The protrusions 42A are continuously arranged in the circumferential direction of the drum 26A (See Fig. 4) and distanced one from another by a predetermined dimension in an axial direction of the drum 26A so that a space 49 of the molding depression 38 is defined between each pair of the adjacent protrusions 42A (See Fig. 3). Alternatively, the protrusions 42A may be intermittently arranged, i.e., distanced one from another in the circumferential direction of the drum 26A. As will be seen in Fig. 4, the protrusion 42A has a triangular profile defined by a pointed apex region 43 and first and second oblique lines 44, 45 (or first and second oblique regions) extending on both sides of the apex region 43 as viewed in the circumferential direction, The apex region 43 lies at a protruding height U1 (i.e., a height dimension) as measured from the bottom 40 of the molding depression 38 and this protrusion height U1 is larger than a depressed depth U2 as measured from the peripheral surface 37 of the drum 26A (i.e., a height dimension as measured from the bottom 40 of the molding depression 38 to the peripheral surface 37). In other words, the apex 43 extends outward slightly beyond the peripheral surface 37 of the drum 26A.

The first oblique region 44 lies in front of the apex region 43 as viewed in the circumferential direction and describes a slope obliquely extending from the apex region 43 to the bottom 40 of the molding depression 38. The second oblique region 45 lies behind the apex region 43 as viewed in the circumferential direction and describes a slope obliquely extending from the apex region 43 to the bottom 40 of the molding depression 38. In each pair of the circumferentially adjacent protrusions 42A, 42A, the first oblique region 44 of the one protrusion 42A is opposed to the second oblique region 45 of the other protrusion 42A. In each pair of the axially adjacent protrusions 42A, 42A, the first oblique region 44 of the one protrusion 42A is opposed to the second oblique region 45 of the other protrusion 42A so that each of the protrusions 42A in one row is exposed between each pair of the circumferentially adjacent protrusions 42A arranged in the axially adjacent row. Namely, these protrusions 42A are arranged in a staggering pattern as viewed in the axial direction of the molding drum 26A.

The conveyor 27 is formed with a plurality of fine vent holes (not shown). These vent holes are dimensioned to be permeable for air but not for the mixture 24. There is provided a suction unit 46 below the conveyor 27 so as to be opposed to the molding drum 26A with interposition of the conveyor 27. The conveyor 27 runs in the direction indicated by an arrow at a velocity substantially corresponding to a rotating velocity at which the molding drum 26A and the compression drum 28 rotate. The suction unit 46 sucks air downward from the upper surface of the conveyor 27 through the vent holes. The compression drum 28 is held in nearly contact with the upper surface of the conveyor 27 during rotation around its shaft 47 in the direction indicated by an arrow. Rotation of the drums 26A, 28, the roller 33 and the grinder mill 32 as well as running of the conveyor 27 are effectuated by a driving force of a rotating machine (not shown).

Now a sequence in which the liquid-absorbent material 24A is made using this apparatus 20 for making the absorbent material will be described hereunder. A pulp sheet 48 is fed into a nip defined between a pair of the guide rollers 33 opposed to each other. The pulp sheet 48 is guided by these guide rollers 33 into the grinder mill 32 and crushed or disintegrated therein into fluff pulp 21. Then the fluff pulp 21 is transferred from the grinder mill 32 into the duct 25. On the other hand, the polymer particles 22 and the thermoplastic synthetic resin fibers 23 are respectively supplied from the hoppers 34, 35 into the duct 25. Within the duct 25, the suction mechanism 39 operatively associated with the molding drum 26A sucks air from the inlet 29 toward the outlet 30 as indicated by an arrow V1. The fluff pulp 21 and the polymer particles 22 and the synthetic resin fiber 23 are fanned in drift within the duct 25 by the air stream and move together the outlet 30. Within the duct 25, the fluff pulp 21 and the polymer particles 22 and the synthetic resin fibers 23 are agitated and mixed by the air stream to form the mixture 24. The mixture 24 is conveyed by the air stream from the outlet 30 of the duct 25 and blown against the peripheral surface 37 of the molding drum 26A.

The molding depressions 38 are periodically opposed to the outlet 30 of the duct 25 as the molding drum 26A is rotated by the shaft 36. As indicated by an arrow V2, air is continuously sucked by the suction mechanism 39 from the molding depression 38 to the interior of the molding drum 26A, so that, when the molding depression 38 is opposed to the outlet 30 of the molding drum 26A, the mixture 24 is collected in the molding depression 38 and accumulated in the molding depression 38 excluding the protrusions 42A. The mixture 24 accumulated in the molding depression 38 in this manner is held within the molding depression 38 under a sucking force of the suction mechanism 39 thereby to be molded into a liquid-absorbent material 24A having a generally hourglass shape and moves, in this state, closer to the conveyor 27 as the molding drum 26A rotates. When the molding depression 38 is opposed to the conveyor 27, the absorbent material 24A is transferred from the molding depression 38 onto the conveyor 27, as indicated by an arrow V3, under a sucking force of the suction unit 46 located below the conveyor 27. In this way, a plurality of the hourglass-shaped absorbent material 24A is arranged on the upper surface of the conveyor 27 in the running direction of the conveyor 27. The absorbent material 24A are conveyed by the conveyor 27 toward the compression drum 28 and successively compressed between the rotating compression drum 28 and the conveyor 27.

In the molding drum 26A, each of the protrusions 42A comprises the apex region 43 and the first and second oblique regions 44, 45 wherein these oblique regions 44, 45 respectively describe the slopes obliquely extending from the apex region 43 toward the bottom 40 of the molding depression 38. With such a configuration of the molding depression 38, the mixture 24 is accumulated on the oblique regions 44, 45 while the apex regions 43 of the respective protrusions 42A cause the absorbent material 24A to be formed with through-holes. Thereafter, with compression of the absorbent material 24A by the compression drum 28, the through-holes may be narrowed or closed to form grooves. Such absorbent material 24A will be described later in more detail.

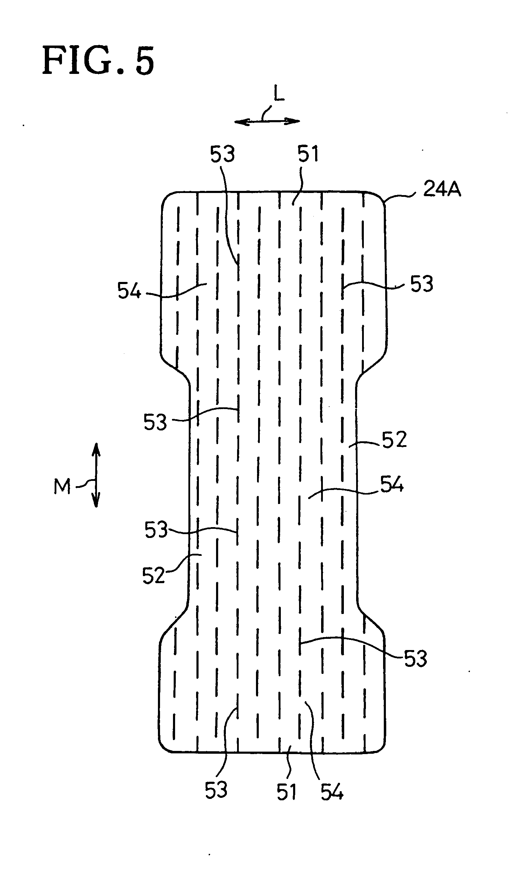

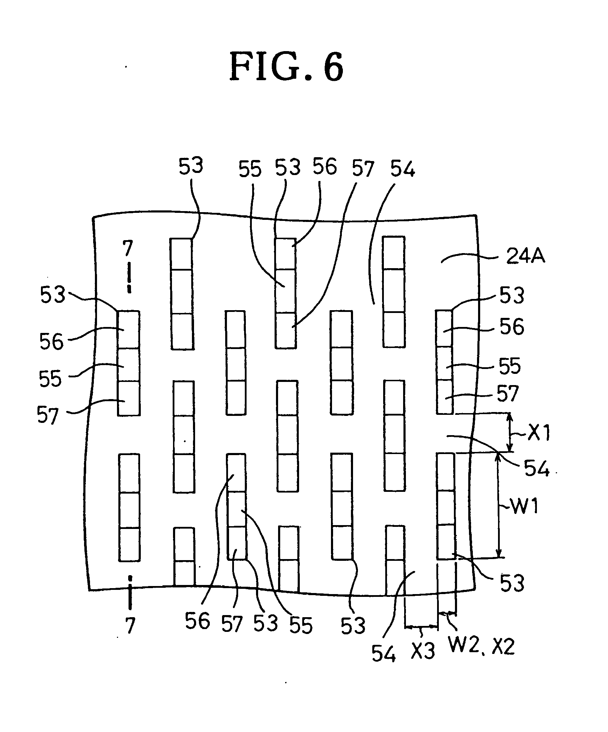

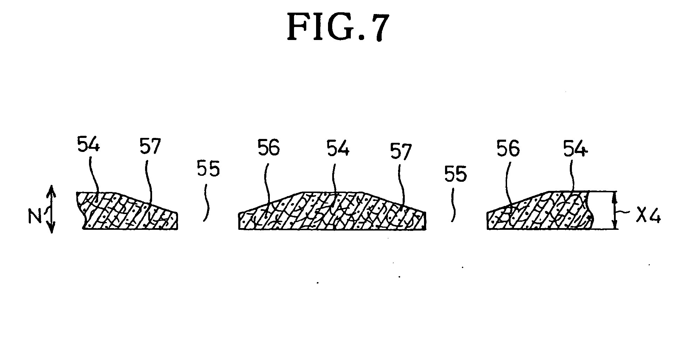

Fig. 5 is a plan view showing the liquid-absorbent material 24A molded using the molding drum 26A shown in Fig. 1, Fig. 6 is a plan view showing a part of Fig. 5 in an enlarged scale and Fig. 7 is a sectional view taken along the line 7-7 in Fig. 6. In Fig. 5, a transverse direction is indicated by an arrow L and a longitudinal direction is indicated by an arrow M. In Fig. 7, a thickness direction is indicated by an arrow N. The liquid-absorbent material 24A presents a generally hourglass planar shape and has longitudinally opposite margins 51 extending in the transverse direction and transversely opposite lateral margins 52 extending in the longitudinal direction. The absorbent material 24A has a plurality of patterned zones 53 which are relatively long in the longitudinal direction and the non-patterned zone 54.

In the absorbent material 24A, the patterned zone 53, the non-patterned zone 54, the patterned zone 53 and the non-patterned zone 54 are arranged in the longitudinal direction in this order, i.e., the patterned zones 53 are arranged intermittently in the longitudinal direction with interposition of the non-patterned zone 54. In the transverse direction, the patterned zone 53, the non-patterned zone 54, the patterned zone 53 and the non-patterned zone 54 are arranged in this order, i.e., the patterned zones 53 are arranged intermittently in the transverse direction with interposition of the non-patterned zone 54. The patterned zones 53 mean the aforementioned narrowed through-holes or closed grooves. A density of the absorbent material 24A in the closed grooves of the patterned zones 53 is less than that of the non-patterned zone 54. This is because the absorbent material 24A, particularly the non-patterned zone 54, is compressed as aforementioned and the closed grooves of the patterned zones 53 have the mixture 24 of a basis weight less than that of the non-patterned zone 53. Consequentially, a stiffness of the absorbent material 24A in the closed grooves is lower than that of the non-patterned zone 54.

The patterned zone 53 has a longitudinal dimension W1 in a range of 10 to 60 mm and a transverse dimension W2 in a range of 1 to 5 mm. The non-patterned zone 54 defined between each pair of the longitudinally adjacent patterned zones 53 has a longitudinal dimension X1 in a range of 10 to 20 mm and a transverse dimension X2 in a range of 1 to 5 mm. The non-patterned zone 54 defined between each pair of the transversely adjacent patterned zones 53 has a transverse dimension X3 in a range of 5 to 25 mm (See Fig. 6). The non-patterned zone 54 has a thickness dimension X4 in a range of 2 to 5 mm (See Fig. 7).

The patterned zone 53 comprises a first segment 55 occupying a middle of this zone 53, and second and third segments 56, 57 lying on both sides of the first segment 55 as viewed in the longitudinal direction. In the patterned zone 53, the second segment 56 lies in front of the first segment 55 and the third segment 57 lies behind the first segment 55 as viewed in the longitudinal direction. In each pair of the longitudinally adjacent patterned zones 53, the second segment 56 of the patterned zone 53 is opposed to the third segment 57 of the other patterned zone 53 with interposition of the non-patterned zone 54. In each pair of the transversely adjacent patterned zones 53, the second segment 56 of the one patterned zone 53 is opposed to the third segment 57 of the other patterned zone 53 with interposition of the non-patterned zone 54. Between the first segments 55 of the transversely adjacent patterned zones 53, the non-patterned zone 54 is interposed and extends between each pair of the longitudinally adjacent patterned zones 53. In this manner, the patterned zones 53 are arranged in a staggering pattern in the transverse direction.

The first segment 55 corresponds to the aforementioned through-hole of the absorbent material 24A in which the absorbent material 24A is devoid or, though not shown in Fig. 7, may be slightly present. The second and third segments 56, 57 form grooves depressed in the thickness direction of the absorbent material 24A and thickness dimension of these segments 56, 57 is smaller than that of the non-patterned zone 54. Therefore, basis weight of the absorbent material 24A in the second and third segments 56, 57 is less than that of the absorbent material 24A in the non-pattern zone 54 and stiffness of the absorbent material 24A in the second and third segments 56, 57 is less than that of the absorbent material 24A in the non-patterned zone 54. Thickness dimension of the absorbent material 24A in the second and third segments 56, 57 is gradually reduced from the non-patterned zone 54 toward the first segment 55 and basis weight of the absorbent material 24A correspondingly is gradually reduced from the non-patterned zone 54 toward the first segment 55.

Due to differential specific gravity of the fluff pulp 21, the polymer particles 22 and the synthetic resin fibers 23 as well as turbulence in the air stream, it is difficult for the molding drum 26A to accumulate the mixture 24 evenly in the molding depression 38 over its whole area. Consequentially, there is a possibility that the mixture 24 might be locally accumulated in the molding depression 38 in a concentrated manner and zones (not shown) in which basis weight of the mixture 24 is relatively high might be locally formed in the molding depression 38. If the absorbent material 24A molded from the mixture 24 including such zones is compressed as it is, the finished absorbent material 24A might be locally formed with high stiffness zones (not shown) having high density. However, even if the high density zones are locally formed in the molding depression 38, such high density zones are segmented by the protrusions 42A since the mixture 24 is accumulated in the molding depression 38 except the protrusions 42A. In this way, the high density zones are segmented by the protrusions 42A and stiffness of the absorbent material 24A in these high density zones is effectively reduced by the protrusions 42A.

As will be apparent from Figs. 5 and 6, the absorbent material 24A as an absorbent core made using this molding drum 26A has a plurality of the patterned zones 53 formed by the protrusions 42A so as to be intermittently arranged. The high stiffness zones are segmented by these patterned zones 53 and stiffness of the high stiffness zones is reduced by these patterned zones 53 even if the absorbent material 24A is locally formed with the high stiffness zones. The high stiffness zones segmented by the patterned zones 53 allow body fluids to be absorbed by the absorbent material over its whole area rather than being absorbed by the high stiffness zones in concentrated manner. In this way, this molding drum 26A is able to make the absorbent material 24A having generally uniform stiffness and high flexibility so that body fluids can be efficiently absorbed by the absorbent material 24A over its whole area.

Of each pair of the protrusion 42A adjacent to each other in the axial direction on the molding drum 26A, the first oblique region 44 of the one protrusion 42A is opposed to the second oblique region 45 of the other protrusion 42A and these protrusions 42A are arranged in staggering pattern as viewed in the axial direction. With such a unique arrangement, the molding depression 38 is segmented by the protrusions 42A to make the molding depression 38 discontinuous in the axial direction. In this way, the high density zones can be reliably segmented by the protrusions 42A and stiffness of the absorbent material 24A in the high density zones can be reliably reduced by these protrusions 42A.

Fig. 8 is a partially cutaway plan view showing a disposable diaper 100 including the absorbent material 24A shown in Fig. 5. In Fig. 8, a transverse direction is indicated by an arrow L and a longitudinal direction is indicated by an arrow M. The diaper 100 comprises a liquid-pervious topsheet 101 facing the wearer's body, a liquid-impervious backsheet 102 facing away from the wearer's body and the absorbent material 24A interposed between these sheets 101, 102. The diaper 100 is contoured by longitudinally opposite margins 103 extending in parallel to each other in the transverse direction and transversely opposite lateral margins 104 extending in the longitudinal direction, defining a front waist region 105, a rear waist region 107 and a crotch region 106 extending between these waist regions 105, 107 arranged in the longitudinal direction. The transversely opposite lateral margins 104 in the crotch region 106 describe circular arcs which are convex inward as viewed in the transverse direction of the diaper 100. The diaper 100 has a generally hourglass-like planar shape.

As a stock material for the topsheet 101, a hydrophilic fibrous nonwoven fabric is used. As a stock material for the backsheet 102, a breathable liquid-impervious plastic film is used. The fibrous nonwoven fabric may be selected from the group consisting of those obtained by spun lace-, needle punch-, melt blown-, thermal bond-, spunbond- and chemical bond-processes. Component of the nonwoven fabric to be used may be selected from the group consisting of polyester-, polyacrylonitorile-, polyvinyl chloride-, polyethylene-, polypropylene- and polystyrene-based fibers. The group from which the appropriate component fibers may be selected further includes core-sheath type composite fibers, juxtaposed type composite fibers, macaroni fibers, microporous fibers and conjugated type composite fibers. A film used in this invention may be an oriented plastic film containing fine particles of inorganic substance such as silica or alumina. The absorbent material 24A is entirely wrapped with a water-pervious sheet 108 such as a tissue paper or hydrophilic fibrous nonwoven fabric in order to prevent the absorbent material 24A from getting out of its initial shape. The absorbent material 24A is laid so as to occupy the front and rear waist regions 105, 107 and the crotch region 106 except the longitudinally opposite margins 103 and the transversely opposite lateral margins 104 and bonded to the inner surfaces of the top- and backsheets 101, 102 by the intermediary of the water-pervious sheet 108.

The longitudinally opposite margins 103 are defined by longitudinally opposite margins 109 of the topsheet 101 and longitudinally opposite margins 110 of the backsheet 102 both extending in the longitudinal direction beyond longitudinally opposite ends 51 of the absorbent material 24A. Along the longitudinally opposite margins 103, the longitudinally opposite margins 109 of the topsheet 101 and the longitudinally opposite margins 110 of the backsheet 102 are put flat together and respectively have the inner surfaces permanently bonded together. The longitudinally opposite margins 103 are provided with waist elastic members 111 extending in the transverse direction contractibly bonded thereto. The waist elastic members 111 are sandwiched between the opposite margins 109 of the topsheet 101 and the opposite margins 110 of the backsheet 102, stretched at a predetermined ratio in the transverse direction and permanently bonded in such stretched state to the respective inner surfaces of these sheets 101, 102.

The opposite lateral margins 104 are defined by transversely opposite lateral margins 112 of the topsheet 101 and transversely opposite lateral margins 113 of the backsheet 102 extending in the transverse direction beyond opposite side edges 52 of the absorbent material 24A. Along these lateral margins 104, the opposite lateral margins 112 of the topsheet 101 and the opposite lateral margins 113 of the backsheet 102 are put flat together and respectively have the inner surfaces permanently bonded together. These lateral margins 104 are provided with a plurality of leg elastic members 114 extending in the longitudinal direction contractibly bonded thereto. The leg elastic members 114 are sandwiched between the opposite lateral margins 112 of the topsheet 101 and the opposite lateral margins 113 of the backsheet 102, stretched at a predetermined ratio in the longitudinal direction and permanently bonded in such stretched state to the respective inner surfaces of these sheets 101, 102.

The opposite lateral margins 104 of the rear waist region 107 are respectively provided with tape fasteners 115 made of a plastic film and attached thereto. Each of the tape fasteners 115 comprises a fixed end 116 and a free end 117 both extending in the transverse direction. The fixed end 116 is sandwiched between the lateral margin 112 of the topsheet 101 and the lateral margin 113 of the backsheet 102 and permanently bonded to respective inner surfaces of these sheets 101, 102. The free end 117 is provided on its inner surface with a male mechanical fastener 118 having a plurality of hooks and attached thereto. The front waist region 105 is provided with a target tape strip 119 attached thereto so that the respective free ends 117 of the tape fasteners 115 may be detachably anchored on this target tape strip 119. As the target tape strip 119, a female mechanical fastener comprising a base and a plurality of loops protruding from this base. The target tape 119 has a rectangular shape which is relatively long in the transverse direction and is permanently bonded to the outer surface of the backsheet 102.

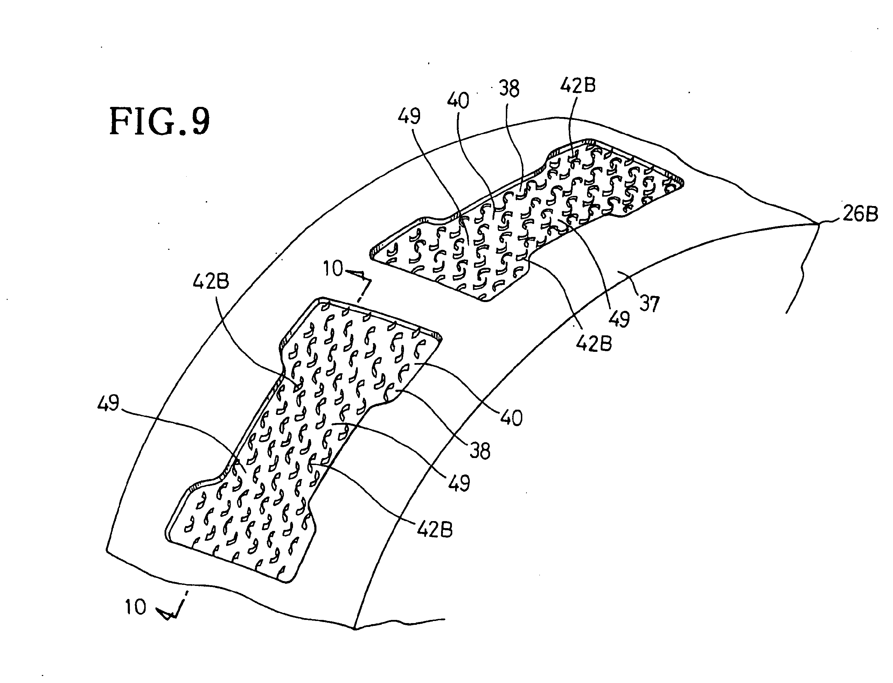

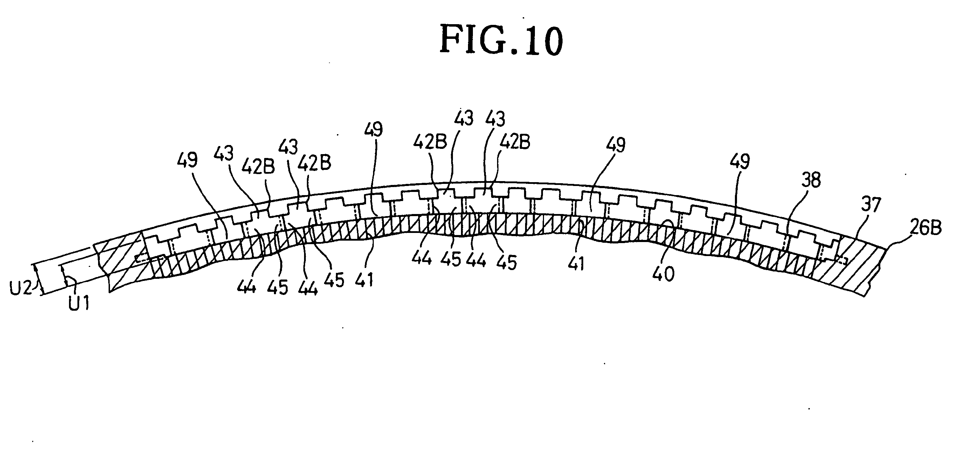

Fig. 9 is a perspective partial view showing, in an enlarged scale, a peripheral surface 37 and a molding depression 38 of a molding drum 26B according to a first example that does not constitute an embodiment of the invention and Fig. 10 is a sectional view taken along the line 10-10 in Fig. 9. This molding drum 26B is similar to that shown in the first embodiment of the invention except that protrusions 42B formed on the bottom 40 of the molding depression 38 respectively describe semi-circular arcs which are convex alternately in axially opposite direction of the molding drum 26B. The other features are the same as those in the molding drum 26A of Fig. 1, so these features are designated by the same reference numerals as those used in the first embodiment of the invention and will not be repetitively described. The apparatus 20 also is similar to that shown in Figs. 1 and 2 of the first embodiment of the invention, so description thereof, if necessary, will be made in reference with Figs. 1 and 2.

Each of the protrusions 42B is relatively long in the circumferential direction of the drum 26B and protrudes outward in a diametrical direction of the drum 26B from a bottom 40 of the molding depression 38. The protrusions 42B are intermittently arranged in the circumferential direction of the drum 26B with interposition of a space 49 (See Fig. 10) and distanced one from another by a predetermined dimension in an axial direction of the drum 26B (See Fig. 9). The protrusions 42B respective describe the semi-circular arcs which are convex in the axial direction of the molding drum 26B. Each pair of the protrusions 42B which are adjacent to each other in the circumferential direction describes the semi-circular arcs which are convex alternately in axially opposite directions. The protrusion 42B has an apex region 43 and first and second regions 44, 45 lying on both sides of the apex region 43 as viewed in the circumferential direction. The apex region 43 lies at a protruding height U1 (i.e., a height dimension) as measured from the bottom 40 of the molding depression 38 and this protrusion height U1 is smaller than a depressed depth U2 as measured from the peripheral surface 37 of the drum 26B (i.e., a height dimension as measured from the bottom 40 of the molding depression 38 to the peripheral surface 37). In other words, the apex 43 and the first and second regions 44, 45 lie inside the peripheral surface 37 of the drum 26B.

The first region 44 lies in front of the apex region 43 as viewed in the circumferential direction and has a protruding height (i.e., height dimension) as measured from the bottom 40 of the molding depression 38 smaller than that of the apex region 43. The second region 45 lies behind the apex region 43 as viewed in the circumferential direction and has a protruding height (i.e., height dimension) smaller than that of the apex region 43. A step is formed between the apex region 43 and the first region 44 as well as between the apex region 43 and the second region 45. In each pair of the protrusions 42B which are adjacent to each other in the circumferential direction, the first region 44 of the one protrusion 42B opposed to the second region 45 of the other protrusion 42B. In each pair of the protrusions 42B which are adjacent to each other in the axial direction, the first region 44 of the one protrusion 42B is opposed to the second region 45 of the other protrusion 42B. In this manner, these protrusions 42B are arranged in staggering pattern in the axial direction of the molding drum 26B.

A sequence in which the liquid-absorbent material is made using this molding drum 26B is similar to the sequence using the apparatus 20 shown in the first embodiment of the invention and description thereof will not be repeated hereunder. However, it should be noted here that the apex regions 43 and the first and second regions 44, 45 of the protrusions 42B lie inside the peripheral surface 37 of the molding drum 26B. Consequentially, the mixture 24 is accumulated not only in the molding depression 38 but also on the apex regions 43 and the first and second regions 44, 45 of the protrusions 42B. The liquid-absorbent core made using this molding drum 26B comprises, as will be described later in more detail, a plurality of semi-circular patterned zones formed by the protrusions 42B and the non-patterned zone formed by the space 49 of the molding depression 38.

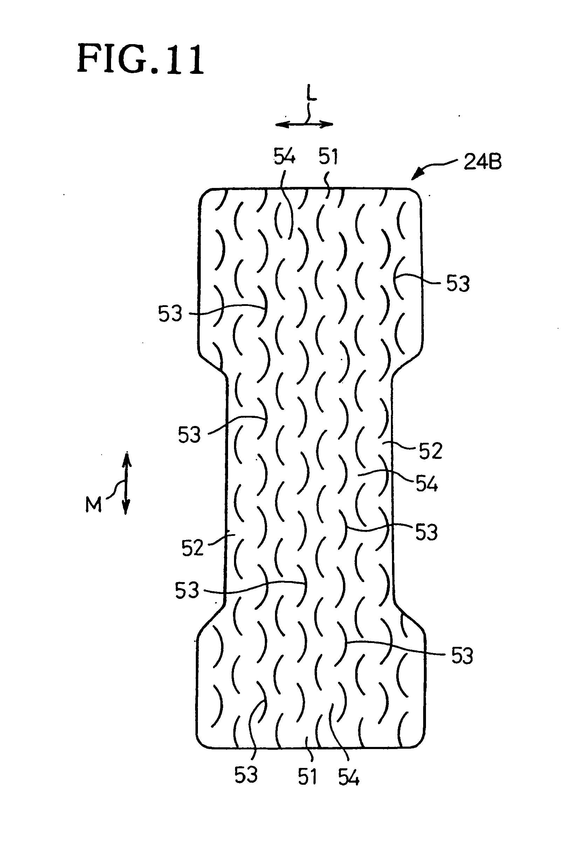

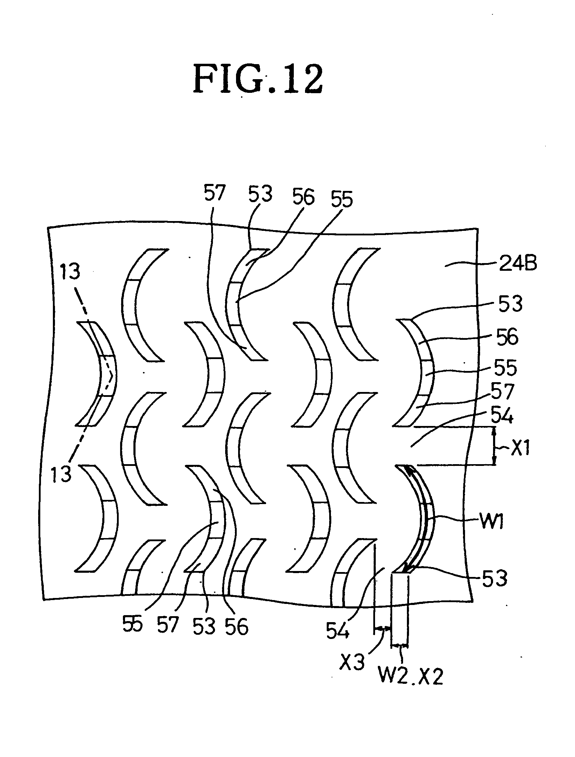

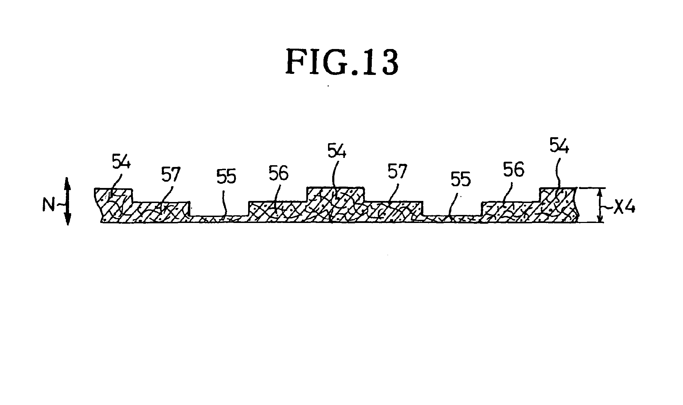

Fig. 11 is a plan view showing the liquid-absorbent material 24B made using the molding drum 26B shown in Fig. 9, Fig. 12 is a plan view showing a part of Fig. 11 in an enlarged scale and Fig. 13 is a sectional view taken along the line 13-13 in Fig. 12. In Fig. 11, a transverse direction is indicated by an arrow L and a longitudinal direction is indicated by an arrow M. In Fig. 13, a thickness direction is indicated by an arrow N. The liquid-absorbent material 24B presents an hourglass-like planar shape and has longitudinally opposite ends 51 extending in the transverse direction and transversely opposite side edges 52 extending in the longitudinal direction. The liquid-absorbent material 24B comprises a plurality of patterned zones 53 which are relatively long in the longitudinal direction and the non-patterned zone 54.

In the liquid-absorbent material 24B, the patterned zone 53, the non-patterned zone 54, the patterned zone 53 and the non-patterned zone 54 are arranged in the longitudinal direction in this order, i.e., the patterned zones 53 are arranged intermittently in the longitudinal direction with interposition of the non-patterned zone 54. In the transverse direction, the patterned zone 53, the non-patterned zone 54, the patterned zone 53 and the non-patterned zone 54 are arranged in this order, i.e., the patterned zones 53 are arranged intermittently in the transverse direction with interposition of the non-patterned zone 54. Density of the absorbent material 24B in the patterned zone 53 is less than that of the non-patterned zone 54. The patterned zones 53 have semi-circular shapes which are convex in the transverse direction wherein each pair of the patterned zones 53 being adjacent to each other in the longitudinal direction describe the semi-circular shapes which are convex alternately in opposite direction. Density of the absorbent material 24B in the patterned zones 53 is less than that of the non-patterned zone. Consequentially, the stiffness of the absorbent material 24B in this patterned zone 53 is lower than in the non-patterned zone 54.

The patterned zone 53 comprises a first segment 55 occupying a middle of this zone 53, and second and third segments 56, 57 lying on both sides of the first segment 55 as viewed in the longitudinal direction. In the patterned zone 53, the second segment 56 lies in front of the first segment 55 and the third segment 57 lies behind the first segment 55 as viewed in the longitudinal direction. In each pair of the longitudinally adjacent patterned zones 53, the second segment 56 of the one patterned zone 53 is opposed to the third segment 57 of the other patterned zone 53 with interposition of the non-patterned zone 54. In each pair of the transversely adjacent patterned zones 53, the second segment 56 of the one patterned zone 53 is opposed to the third segment 57 of the other patterned zone 53 with interposition of the non-patterned zone 54. Between the first segments 55 of the transversely adjacent patterned zones 53, the non-patterned zone 54 is interposed and extends between each pair of the longitudinally adjacent patterned zones 53. In this manner, the patterned zones 53 are arranged in a staggering pattern in the transverse direction.

The first through third segments 55, 56, 57 correspond to grooves depressed in the thickness direction of the liquid-absorbent material 24B. A thickness dimension and basis weight of the second and third segments 55, 56, 57 are smaller than those of the non-patterned zone 54 and larger than those of the first segment 55. Therefore, a stiffness of the second and third segments 56, 57 is less than that of the non-patterned zone 54. A basis weight of the first segment 55 is less than that of the second and third segments 56, 57 and a stiffness of first segments 55 is less than that of the second and third segments 56, 57. This absorbent material 24B is incorporated in the disposable body fluid absorbent article such as the disposable diaper 100 shown in Fig. 8, a sanitary napkin or pad for incontinent patient.

With this molding drum 26B, high density zones in which the absorbent material 24B has a high density may be locally formed in the molding depression 38 and, as a result, the finished absorbent material 24B may be locally formed with high stiffness zones (not shown) in which a stiffness of the absorbent material 24B is relatively high. However, even if the high density zones in which the absorbent material 24B has a relatively high density being locally formed in the molding depression 38, such high density zones are segmented by the protrusions 42A since the mixture 24 is primarily accumulated in the molding depression 38 except the protrusions 42B. In this way, these high density zones are segmented by the protrusions 42B and a density these high density zones is effectively reduced by the protrusions 42B.

As will be apparent from Figs. 12 and 13, the absorbent material 24B made using this molding drum 26B has a plurality of the semi-circular patterned zones 53 formed by the protrusions 42B so as to be intermittently arranged. The high stiffness zones are segmented by these patterned zones 53 and a stiffness of the high stiffness zones is reduced by these patterned zones 53 even if the absorbent material 24B is locally formed with the high stiffness zones. The high stiffness zones segmented by the patterned zones 53 allow body fluids to be absorbed by the absorbent material 24B over its whole area rather than being absorbed by the high stiffness zones in concentrated manner. In this way, this molding drum 26A is able to make the absorbent material 24B having a generally uniform stiffness and high flexibility so that body fluids can be efficiently absorbed by the absorbent material 24B over its whole area.

Of each pair of the protrusion 42B adjacent to each other in the axial direction on the molding drum 26B, the first region 44 of the one protrusion 42B is opposed to the second region 45 of the other protrusion 42B and these protrusions 42B are arranged in staggering pattern as viewed in the axial direction. With such a unique arrangement, the molding depression 38 is segmented by the protrusions 42B to make the molding depression 38 discontinuous in the axial direction and the high density zones can be reliably segmented by the protrusions 42B. In the case of this molding drum 26B, the protrusions 42B have the semi-circular shapes describing circular arcs which are convex in the axial direction of the molding drum 26B and each pair of the circumferentially adjacent protrusions 42B describe the circular arcs being convex alternately in axially opposite directions. Such an arrangement advantageously results in differential distance between each pair of the protrusions 42B being adjacent in the axial direction and generation of turbulence around the protrusions 42B. This turbulence agitates the absorbent material 24B and evenly disperse the fluff pulp, polymer particles and synthetic resin fibers constituting the absorbent material 24B around the protrusions 42B.

The molding drum 26B of Fig. 9 may be alternatively constructed without departing from the scope of the invention in such a manner that the protruding height of the apex region 43 as measured from the bottom 40 of the molding depression 38 is larger than the depth of the molding depression 38 as measured from the peripheral surface 37 of the molding drum 26B and therefore the apex region 43 protrudes outward slightly beyond the peripheral surface 37. In the apparatus 20, it is also possible without departing from the scope of the invention to eliminate the hopper 35 adapted to supply the synthetic resin fibers. In this case, the mixture 24 consists of the fluff pulp and the super-absorbent polymer particles.

## Claims

1. A liquid-absorbent material molding drum (26A) comprising:
a peripheral surface (37) extending in a circumferential direction (A);
a plurality of molding depressions (38) formed on said peripheral surface;
a suction mechanism (39) adapted to suck air inward with respect to said circumferential direction from vent holes formed in a bottom of said molding drum; and
said molding depression being formed on said bottom with a plurality of protrusions (42A), each protruding outward in a diametrical direction of said molding drum and being relatively long in said circumferential direction of said molding drum, said plurality of protrusions being continuously or intermittently arranged in said circumferential direction but distanced one from another by a predetermined dimension in an axial direction of said molding drum,
**characterized in that** a height dimension of an apex region of each protrusion as measured from the bottom of said molding depression is larger than a height dimension as measured from the bottom of said molding depression to said peripheral surface, such that said apex region protrudes outward beyond said peripheral surface.

2. The molding drum as defined by Claim 1, wherein the apex region (43) of each of said protrusions lies in a longitudinal middle of said protrusion, a first region (44) lying in front of said apex as viewed in said circumferential direction and defining a slope obliquely extending from said apex region toward the bottom of said molding depression, and a second region (45) lying behind said apex region as viewed in said circumferential direction and defining a slope obliquely extending from said apex region toward the bottom of said molding depression.

3. The molding drum defined by Claim 2, wherein, in each pair of said protrusions adjacent to each other in said circumferential direction, the first region of the one protrusion is opposed to the second region of the other protrusion and, in each pair of the protrusions adjacent to each other in said axial direction, the first region of the one protrusion is opposed to the second region of the other protrusion.

4. The molding drum as defined by any preceding claim, wherein said protrusion describes a semi-circular arc being convex in said axial direction.

5. The molding drum as defined by Claim 4, wherein each pair of said protrusions being adjacent to each other in said circumferential direction describe the semi-circular arcs which are convex alternately in axially opposite directions.

## Patentansprüche

1. Trommel (26A) zum Formen von flüssigkeitabsorbierendem Material, mit
einer Umfangsfläche (37), die in einer Umfangsrichtung (A) verläuft,
mehreren Formmulden (38), die auf der Umfangsfläche gebildet sind,
einem Ansaugmechanismus (39), der dazu ausgelegt ist, Luft von in einem Boden der Formtrommel gebildeten Lüftungslöchern bezüglich der Umfangsrichtung nach innen zu saugen,
wobei die in dem Boden gebildeten Formmulden mehrere Vorsprünge (42A) aufweisen, die jeweils in einer diametralen Richtung der Formtrommel nach außen ragen und in der Umfangsrichtung der Formtrommel relativ lang sind, wobei die mehreren Vorsprünge kontinuierlich oder periodisch in der Umfangsrichtung angeordnet sind, aber in einer axialen Richtung der Formtrommel einen vorbestimmten Abstand voneinander aufweisen,
**dadurch gekennzeichnet, daß** eine Höhenerstreckung eines Spitzenabschnitts jedes Vorsprungs gemessen von dem Boden der Formmulde größer ist als eine Höhenerstreckung gemessen von dem Boden der Formmulde zu der Umfangsfläche, so daß der Spitzenabschnitt nach außen über die Umfangsfläche hinausragt.

2. Formtrommel nach Anspruch 1, wobei der Spitzenabschnitt (43) jedes der Vorsprünge in einem longitudinalen Mittelbereich des Vorsprungs liegt, ein erster Abschnitt (44) in der Umfangsrichtung gesehen vor dem Spitzenabschnitt liegt und eine schräg von dem Spitzenabschnitt zu dem Boden der Formmulde verlaufende Steigung definiert, und ein zweiter Abschnitt (45) in der Umfangsrichtung gesehen hinter dem Spitzenabschnitt liegt und eine von dem Spitzenabschnitt zu dem Boden der Formmulde schräg verlaufende Steigung definiert.

3. Formtrommel nach Anspruch 2, wobei in jedem Paar von in der Umfangsrichtung aneinander angrenzenden Vorsprüngen der erste Bereich des einen Vorsprungs entgegengesetzt zu dem zweiten Bereich des anderen Vorsprungs liegt, und in jedem Paar von in der Axialrichtung aneinander angrenzenden Vorsprüngen der erste Bereich des einen Vorsprungs entgegengesetzt zu dem zweiten Bereich des anderen Vorsprungs liegt.

4. Formtrommel nach einem der vorstehenden Ansprüche, wobei der Vorsprung einen halbkreisförmigen Bogen beschreibt, der in der Axialrichtung konvex ist.

5. Formtrommel nach Anspruch 4, wobei jedes Paar von in der Umfangsrichtung aneinander angrenzenden Vorsprüngen halbkreisförmige Bögen beschreibt, die in axial entgegengesetzten Richtungen abwechselnd konvex sind.

## Revendications

1. Tambour de moulage de matériau absorbant les liquides (26A), comprenant :
une surface périphérique (37) s'étendant dans une direction circonférentielle (A),
une pluralité d'évidements de moulage (38) formés sur ladite surface périphérique,
un mécanisme d'aspiration (39) conçu pour aspirer l'air vers l'intérieur par rapport à ladite direction circonférentielle à partir de trous d'évent formés en fond dudit tambour de moulage, et
lesdits évidements de moulage étant formés sur ledit fond avec une pluralité de protubérances (42A), chacune dépassant vers l'extérieur dans une direction diamétrale dudit tambour de moulage et étant relativement longue dans ladite direction circonférentielle dudit tambour de moulage, ladite pluralité de protubérances étant agencée en continu ou par intermittence dans ladite direction circonférentielle mais étant espacées les unes des autres d'une dimension prédéterminée dans une direction axiale dudit tambour de moulage,
**caractérisé en ce que** la dimension en hauteur d'une région de sommet de chaque protubérance telle que mesurée à partir du fond dudit évidement de moulage, est supérieure à la dimension en hauteur telle que mesurée à partir du fond dudit évidement de moulage jusqu'à ladite surface périphérique, de telle sorte que ladite région de sommet dépasse vers l'extérieur au-delà de ladite surface périphérique.

2. Tambour de moulage selon la revendication 1, dans lequel la région de sommet (43) de chacune desdites protubérances se trouve sur une partie médiane longitudinale de ladite protubérance, une première région (44) se trouvant devant ledit sommet, comme observé dans ladite direction circonférentielle, et définissant une pente s'étendant obliquement à partir de ladite région de sommet vers le fond dudit évidement de moulage, et une deuxième région (45) se trouvant derrière ladite région de sommet, comme observé dans ladite direction circonférentielle, et définissant une pente s'étendant obliquement à partir de ladite région de sommet vers le fond dudit évidement de moulage.

3. Tambour de moulage selon la revendication 2, dans lequel, dans chaque paire desdites protubérances adjacentes les unes aux autres dans ladite direction circonférentielle, la première région de la première protubérance est opposée à la deuxième région de l'autre protubérance et, dans chaque paire de protubérances adjacentes les unes aux autres dans ladite direction axiale, la première région de la première protubérance est opposée à la deuxième région de l'autre protubérance.

4. Tambour de moulage selon l'une quelconque des revendications précédentes, dans lequel ladite protubérance décrit un arc demi-circulaire qui est convexe dans ladite direction axiale.

5. Tambour de moulage selon la revendication 4, dans lequel chaque paire desdites protubérances, qui sont adjacentes les unes aux autres dans ladite direction circonférentielle, décrit des arcs demi-circulaires qui sont convexes en alternance dans des directions axialement opposées.
